# EUROPEAN PATENT SPECIFICATION

(11) **EP 1 113 818 B1**
(45) Date of publication and mention of the grant of the patent: **17.05.2006**
(21) Application number: 99948333.2
(22) Date of filing: 17.09.1999
(51) Int. Cl.: A61K 39/395, A61K 39/35, A61K 39/39

(54) **METHODS OF TREATING IgE-ASSOCIATED DISORDERS AND COMPOSITIONS FOR USE THEREIN**
VERFAHREN ZUR BEHANDLUNG VON IG-E ASSOZIERTEN KRANKHEITEN UND ZUSAMMENSETZUNGEN ZUR VERWENDUNG IN DIESEN VERFAHREN
PROCEDES DE TRAITEMENT DE TROUBLES ASSOCIES A IgE ET COMPOSITIONS POUVANT ETRE UTILISEES A CET EFFET

(30) Priority: 18.09.1998 US 100838 P; 28.05.1999 US 136600 P; 16.09.1999 US 397198
(43) Date of publication of application: 11.07.2001
(60) Divisional application: 05024789.9
(73) Proprietor: Dynavax Technologies Corporation, Berkeley, CA 94710 (US)
(72) Inventor: DINA, Dino, Oakland, CA 94618 (US)
(74) Representative: Roques, Sarah Elizabeth
(86) International application number: PCT/US1999/021686
(87) International publication number: WO 2000/016804

(56) References cited:
- WO-A-92/21031
- WO-A-97/42947
- HEUSSER C ET AL: "Therapeutic potential of anti-IgE antibodies." CURRENT OPINION IN IMMUNOLOGY, (1997 DEC) 9 (6) 805-13. REF: 76 , XP002125679
- HELLMAN L. ET AL: "Allergy vaccines: A review of developments." CLINICAL IMMUNOTHERAPEUTICS, (1996) 6/2 (130-142). , XP000857620
- VALENTA R ET AL: "The immunoglobulin E-allergen interaction: a target for therapy of type I allergic diseases." INTERNATIONAL ARCHIVES OF ALLERGY AND IMMUNOLOGY, (1998 JUL) 116 (3) 167-76. REF: 64 , XP000857685
- SPIEGELBERG, H.L. ET AL: "Inhibition of IgE formation and allergic inflammation by allergen gene immunization and by CpG motif immunostimulatory oligodeoxynucleotides" ALLERGY [ALLERGY], (SEPTEMBER 1998) VOL. 53, PP. 93-97. ISSN: 0105-4538., XP000857686 Department of Pediatrics, 0833 University of California San Diego, School of Medicine, 9500 Gilman Drive, La Jolla CA 92093-0833, USA

## Description

### TECHNICAL FIELD

The present invention provides methods of treating IgE-associated disorders and compositions for use therein. The methods are particularly useful in treatment of allergies and allergy-related disorders.

### BACKGROUND ART

Allergic responses, including those of allergic asthma and allergic rhinitis, are characterized by an early phase response, which occurs within seconds to minutes of allergen exposure and is characterized by cellular degranulation, and a late phase response, which occurs 4 to 24 hours later and is characterized by infiltration of eosinophils into the site of allergen exposure. Specifically, during the early phase of the allergic response, activation of Th2-type lymphocytes stimulates the production of antigen-specific IgE antibodies, which in turn triggers the release of histamine and other mediators of inflammation from mast cells and basophils. During the late phase response, IL-4 and IL-5 production by CD4⁺ Th2 cells is elevated. These cytokines appear to play a significant role in recruiting eosinophils into the site of allergen exposure, where tissue damage and dysfunction result.

Currently, antigen immunotherapy for allergic disorders involves the subcutaneous injection of small, but gradually, increasing amounts, of antigen in a process called desensitization therapy. Such immunotherapy generally consists of many injections over the course of months, followed by maintenance therapy with generally monthly injections over the course of up to five years, with no laboratory indicators to guide discontinuation of therapy. Antigen immunotherapy is merely palliative and, at present, not curative. Weber (1997) *JAMA* 278:1881-1887; Stevens (1998) *Acta Clinica Beligica* 53:66-72; and Canadian Society of Allergy and Clinical Immunology (1995) *Can. Med. Assoc. J.* 152:1413-1419. Many patients who begin the therapy do not complete the regimen, and-if injections are missed for over a week the patient must begin the entire treatment regimen again. A variety of antigens have been identified and produced by recombinant means. For reviews, see Baldo et al. Allergy (1989), 44:81-97; Baldo Curr Opin Immunol. (1991), 3:841-50; Blaser Ther Umsch (1994), 51:19-23; Bousquet Arb Paul Ehrlich Inst Bundesamt Sera Impfstoffe Frankf A K 1994:257-62; Bousquet et al. Adv Exp Med Biol. (1996), 409:463-9; Breiteneder et al. Arb Paul Ehrlich Inst Bundesamt Sera Impfstoffe Frankf A M, 1997:80-6; Crameri et al. Pneumologie (1996), 50 (6):387-93; Donovan et al. Monogr Allergy (1990), 28:52-83; Kraft, Adv Exp Med Biol. (1996), 409:471-4; Nakagawa et al. Int Arch Allergy Immunol. (1993), 102:117-20; Schou, Adv Exp Med Biol. (1996), 409:13 7-40; Thomas, Adv Exp Med Biol. (1996), 409:85-93; Valenta et al. Adv Exp Med Biol. (1996), 409:185-96; Valenta et al. Arb Paul Ehrlich Inst Bundesamt Sera Impfstoffe Frankf A M, 1997:222-9.

Antigen immunotherapy treatments present the risk of inducing potentially lethal IgE-mediated anaphylaxis and do not address the cytokine-mediated events of the allergic late phase response. In fact, one practitioner has described this therapy as having "the potential for misadventure." Weber (1997). Another significant problem with antigen immunotherapy is that the risk of adverse reactions, especially anaphylaxis, significantly reduces the dosage of antigen both with respect to the amount given per administration and the amount given over a period of time. Thus, traditional allergy immunotherapy is protracted and thus time-consuming, inconvenient, and expensive.

An alternative approach for treatment of IgE-associated disorders such as allergies involves administration of compounds which inhibit histamine release. Many such drugs are available as over-the-counter remedies. Other drugs include an anti-IgE binding antibody. However, a drawback of this approach is that it merely masks the symptoms, while not providing any kind of permanent cure or protection.

What is needed are improved methods of treating IgE-associated disorders, such as allergy.

### DISCLOSURE OF THE INVENTION

According to one aspect of the present invention, there is provided a first composition that inhibits the activity of IgE sufficient to decrease the activity of IgE in a subject wherein the first composition comprises an anti-IgE antibody and a second composition comprising an amount of an antigen sufficient to modulate the immune response to the antigen wherein the antigen is linked to a polynucleotide comprising an immunostimulatory oligonucleotide, for use in combination in treating an allergic response to the antigen or allergy-related disorder during antigen-specific immunotherapy of the subject.

Thus, we describe methods of treating an allergic response to an antigen or allergy-related disorder during antigen-specific immunotherapy of a subject by administering to the subject an amount of a first composition that inhibits the activity of IgE sufficient to decrease the activity of IgE in the subject and administering to the subject an amount of the antigen (or a second composition comprising the antigen) sufficient to palliate the response or disorder, wherein the antigen is linked to a polynucleotide comprising an immonustimulatory oligonucleotide.

According to another aspect of the present invention, there is provided a first composition that inhibits the activity of IgE sufficient to palliate an IgE associated disorder wherein the first composition comprises an anti-IgE antibody and a second composition comprising an immunostimulatory oligonucleotide sufficient to augment the activity of the first composition, for use in combination in treating a subject having the IgE associated disorder.

Thus, we also describe methods of treating a subject having an IgE associated disorder by administering to the subject an amount of a first composition that inhibits the activity of IgE sufficient to palliate the disorder and; administering to the subject an amount of a second composition comprising an immunostimulatory oligonucleotide sufficient to augment the activity of the first composition. In the practice of the invention, the first and second compositions can be administered to the subject in any order and simultaneously. Further, as would be readily understood by one skilled in the art, the active ingredients described in any of the embodiments herein (i.e. IgE and/or anaphylaxis inhibitor, ISS, and/or antigen) may be combined into a single composition for simultaneous administration of one or more of the active ingredient(s).

In accordance with a further aspect of the present invention, there is provided a first composition comprising an agent that inhibits anaphylaxis in an amount effective to inhibit anaphylaxis wherein the first composition comprises an anti-IgE antibody and a second composition comprising a polynucleotide comprising an immunostimulatory oligonucleotide in an amount sufficient to enhance the activity of the first composition, for use in combination in treating an IgE-associated disorder in an individual.

We also describe methods of treating an allergic response or allergy-related disorder to an allergen during antigen-specific immunotherapy by administering to the subject an amount of a first composition that inhibits the activity of IgE sufficient to palliate the disorder; administering to the subject an amount of a second composition comprising an immunostimulatory oligonucleotide sufficient to augment the activity of the first composition and; administering to the subject a third composition comprising an amount of the antigen sufficient to induce desensitization to the allergen. Preferably, the third composition is administered after the first composition, so as to avoid anaphylactic shock. Otherwise, the second and third compositions can be administered in any order or simultaneously. The ISS-antigen conjugates described herein are particularly useful for simultaneous administration of these compositions.

The methods provided herein are suitable for treating any IgE associated disorder including, but not limited to allergies, allergy-related disorders and parasite infections. A non-exhaustive list of substances to which subjects can be allergic is provided in Table 1. A variety of allergy-related disorders are known, including, but not limited to, asthma, urticaria and others described herein. Parasite infections include, but are not limited to those associated with helminths.

As described herein, the composition that inhibits the activity of IgE is an anti-IgE antibody.

The invention further provides compositions containing at least one antigen for use in immunotherapy according to the methods described herein. Importantly, these compositions are suitable for administration to a subject in a concentration higher than acceptable for use in allergy desensitization therapy. The composition can thus be supplied in a more concentrated form compared to compositions typically used in desensitization therapy.

The present invention further provides compositions containing a both a composition that inhibits the activity of IgE, comprising an anti-IgE antibody and an immunostimulatory oligonucleotide for use in the methods described herein.

### MODES FOR CARRYING OUT THE INVENTION

This invention provides a combination of (a) one (or more) agent(s) in one or more compositions that inhibits IgE activity in a subject, preferably sufficient to reduce, or block, adverse reactions, including anaphylaxis (upon administration and/or exposure to antigen), and (b) immunotherapy which employs an immunostimulatory polynucleotide or antigen and immunostimulatory polynucleotide sequences (ISS) in the context of IgE-associated disorders, such as allergic conditions. Such a combination allows significant advantages over current, traditional therapy. With the combination therapy of the invention, more aggressive, and therefore more effective, immunotherapy is possible (due to higher amounts of antigen that may be administered) with significantly reduced risk of unwanted side effects, such as anaphylaxis. Further, treatment can proceed more swiftly, saving time and inconvenience. This is a significant consideration especially in traditional allergy immunotherapy, where a patient has to come into a clinic many times over a long period of time, often to only achieve questionable results. The shortened time of therapy also is advantageous because success of treatment can be more readily and accurately determined.

In contrast to conventional desensitization therapy, which maintains a relatively low dosage which slowly increases over time, the present invention permits one to administer antigen in significantly higher doses (i.e., one can administer an amount of antigen that would normally produce a high risk of anaphylaxis (in the absence of administering an IgE inhibitor(s)).

### General Techniques

The practice of the present invention will employ, unless otherwise indicated, conventional techniques of molecular biology (including recombinant techniques), microbiology, cell biology, biochemistry and immunology, which are within the skill of the art. Such techniques are explained fully in the literature, such as, *Molecular Cloning: A Laboratory Manual*, second edition (Sambrook et al., 1989); *Oligonucleotide Synthesis* (M.J. Gait, ed., 1984); *Animal Cell Culture* (R.I. Freshney, ed., 1987); *Methods in Enzymology* (Academic Press, Inc.); *Handbook of Experimental Immunology* (D.M. Weir & C.C. Blackwell, eds.); *Gene Transfer Vectors for Mammalian Cells* (J.M. Miller & M.P. Calos, eds., 1987); *Current Protocols in Molecular Biology* (F.M. Ausubel et al., eds., 1987); *PCR: The Polymerase Chain Reaction,* (Mullis et al., eds., 1994); and *Current Protocols in Immunology* (J.E. Coligan et al., eds., 1991); *The Immunoassay Handbook* (David Wild, ed., Stockton Press NY, 1994); and *Methods of Immunological Analysis* (R. Masseyeff, W.H. Albert, and N.A. Staines, eds., Weinheim: VCH Verlags gesellschaft mbH, 1993).

### Definitions

As used herein, "treatment" is an approach for obtaining beneficial or desired clinical results. For purposes of this invention, beneficial or desired clinical results include, but are not limited to, alleviation of one or more symptoms, diminishment of extent of disorder or disease, stabilized (i.e., not worsening) state of disorder or disease, delay or slowing of disorder or disease progression, amelioration or palliation of the disorder or the disease state, and remission (whether partial or total), whether detectable or undetectable. "Treatment" can also mean prolonging survival as compared to expected survival if not receiving treatment.

"Palliating" a disease or disorder means that the extent and/or undesirable clinical manifestations of a disorder or a disease state are lessened and/or time course of the progression is slowed or lengthened, as compared to not treating the disorder. Especially in the allergy context, as is well understood by those skilled in the art, palliation may occur upon modulation of the immune response against an allergen(s). Further, palliation does not necessarily occur by administration of one dose, but often occurs upon administration of a series of doses. Thus, an amount sufficient to palliate a response or disorder may be administered in one or more administrations.

A "subject" is a vertebrate, preferably a mammal, more preferably a human. Mammals include, but are not limited to, farm animals, sport animals, rodents and pets.

An "IgE associated disorder" is a physiological condition which is characterized, in part, by elevated IgE levels, which may or may not be persistent. IgE associated disorders include, but are not limited to, allergy and allergic reactions, allergy-related disorders (described below), asthma, rhinitis, conjunctivitis, urticaria, shock, hymenoptera sting allergies, and drug allergies, and parasite infections. The term also includes related manifestations of these disorders. Generally, IgE in such disorders is antigen-specific.

An "allergic response to antigen" means an immune response generally characterized by the generation of antigen-specific IgE and the resultant effects of the IgE antibodies. As is well-known in the art, IgE binds to IgE receptors on mast cells and basophils. Upon later exposure to the antigen recognized by the IgE, the antigen crosslinks the IgE on the mast cells and basophils causing degranulation of these cells. It is understood and intended that the terms "allergic response to antigen", "allergy", and "allergic condition" are equally appropriate for application of the methods of the invention. Further, it is understood and intended that the methods of the invention are equally appropriate for prevention of an allergic response as well as treating a pre-existing allergic condition.

An "allergy-related disorder" means a disorder resulting from the effects of an antigen-specific IgE immune response. Such effects can include, but are not limited to, hypotension and shock. Anaphylaxis is an example of an allergy-related disorder during which histamine released into the circulation causes vasodilation as well as increased permeability of the capillaries with resultant marked loss of plasma from the circulation. Anaphylaxis can occur systemically, with the associated effects experienced over the entire body, and it can occur locally, with the reaction limited to a specific target tissue or organ.

A "parasite infection" means infection by metazoan parasites, including helminths, for instance, *Schistosoma mansoni.* For purposes of this invention, a parasitic infection is accompanied by elevated IgE levels (and is thus an IgE associated disorder).

As used herein, the term "antigen" means a substance that is recognized and bound specifically by an antibody or by a T cell antigen receptor. Antigens can include peptides, proteins, glycoproteins, polysaccharides, gangliosides and lipids; portions thereof and combinations thereof. The antigens can be those found in nature or can be synthetic. Haptens are included within the scope of "antigen." A hapten is a low molecular weight compound that is not immunogenic by itself but is rendered immunogenic when conjugated with an immunogenic molecule containing antigenic determinants.

As used herein, the term "allergen" means an antigen or antigenic portion of a molecule, usually a protein, which elicits an allergic response upon exposure to a subject. Typically the subject is allergic to the allergen as indicated, for instance, by the wheal and flare test or any method known in the art. A molecule is said to be an allergen if only a small subset of subjects exhibit an immune response upon exposure to the molecule. A number of isolated allergens are known in the art. These include, but are not limited to, those provided in Table 1 (below).

The term "desensitization" refers to the process of the administration of increasing doses of an allergen to which the subject has demonstrated sensitivity. Examples of allergen doses used for desensitization are known in the art, see, for example, Fornadley (1998) *Otolaryngol. Clin. North Am.* 31:111-127.

An "effective amount" of a substance is that amount sufficient to effect beneficial or desired clinical results, and, as such, an "effective amount" depends upon the context in which it is being applied. In the treatment, or therapy, context, an "effective amount" is an amount sufficient to achieve amelioration or palliation of the disorder being treated by the methods of the present invention. In the context of administering a composition that inhibits IgE activity (i.e., a composition comprising an agent that inhibits IgE), an effective amount is an amount sufficient to achieve such inhibition (which need not be total, or absolute). Most preferably, particularly in the allergy context, an effective amount is an amount sufficient to reduce and/or suppress anaphylaxis (or another unwanted side - effect(s)) upon administration and/or exposure to antigen. An effective amount can be administered in one or more administrations, and it is understood that, especially in the context of allergy desensitization therapy, an effective amount is achieved over a series of administrations, typically in increasing dosages.

The term "ISS" as used herein refers to oligonucleotide sequences that effect a measurable immune response as measured *in vitro, in vivo* and/or *ex vivo.* As is well understood in the art, the term "oligonucleotide" encompasses varying sequence lengths, and as described herein, and readily apparent to those skilled in the art, the term "ISS" includes polynucleotides which contain (or alternatively can consist of) an ISS. Examples of measurable immune responses include, but are not limited to, antigen-specific antibody production, secretion of cytokines, activation or expansion of lymphocyte populations such as NK cells, CD4⁺ T lymphocytes, CD8⁺ T lymphocytes, B lymphocytes, and the like. Preferably, the ISS sequences preferentially activate a Th1-type response. Further description of ISS suitable for use in the present invention are discussed below.

An "ISS-antigen conjugate" refers to a an ISS oligonucleotide or polynucleotide comprising an ISS linked to an antigen through covalent and/or non-covalent interactions.

As used herein, the term "agent" means a biological or chemical compound such as a simple or complex organic or inorganic molecule, a peptide, a protein or an oligonucleotide. A vast array of compounds can be synthesized, for example, proteins, peptides, and polynucleotides, and synthetic organic compounds based on various core structures, and these are also included in the term "agent". In addition, various natural sources can provide compounds, such as plant or animal extracts, and the like. Agents can be administered alone or in various combinations.

A composition or agent which "inhibits IgE activity" is a composition that contains an agent(s) that reduces IgE activity when compared to otherwise same conditions, except for the absence of the composition. As is known in the art, IgE activity is generally indicated and measured by the circulating levels of IgE, but can also be indicated and measured by activities associated with IgE function, such as binding to basophils, anaphylaxis, and binding to receptors such as Fc receptors (including high affinity Fc receptors and low affinity receptor, CD23). Any amount of reduction is sufficient. Preferably, if in terms of circulating IgE, the level is at least about 10%, preferably at least about 20%, preferably at least about 40%, more preferably at least about 50%, more preferably at least about 60%, more preferably at least about 75%, more preferably at least about 80%, more preferably at least about 90%, more preferably at least about 95% reduced. Another measure of reduction of IgE activity, and especially pertinent for the present invention, is inhibition of anaphylaxis (or reduction to elimination of risk of anaphylaxis). Agents which inhibit IgE activity are described herein, and include, but are not limited to, anti-IgE antibodies, IgE receptors, anti-IgE receptor antibodies, ligands for IgE receptors.

An agent (or a composition) which "inhibits" anaphylaxis is one which decreases the extent of anaphylaxis which would have occurred under otherwise same conditions, except for the absence of the agent. Accordingly, an agent or composition which inhibits anaphylaxis is one that reduces (or even eliminates) the risk of anaphylaxis. Most typically inhibiting anaphylaxis entails reducing IgE activity (including reducing circulating IgE levels by, for example, binding to IgE); binding to IgE on IgE-secreting B cells; antagonists; affecting events upstream of IgE production; blocking IgE ability to bind to receptor on mast cells (for example, binding to receptor on mast cell without triggering histamine release). For purposes of this invention, any agent that acts upstream of histamine release is acceptable. An agent which acts to inhibit IgE production and/or activity is preferred, as long as the inhibitor(s) does not induce systemic anaphylaxis. Thus, preferably, the inhibitors of IgE activity used in this invention do not result in histamine release from basophils or mast cells.

### Methods of the invention

The invention provides methods for treating allergies as well as other IgE-associated disorders. Accordingly, in one embodiment, the invention provides methods of treating an allergic response to an antigen or an allergy-related disorder during antigen-specific immunotherapy (i.e. methods for antigen-specific immunotherapy, or desensitization therapy) of a subject, comprising (a) administering a first composition that inhibits IgE activity (preferably inhibits or suppresses anaphylaxis); and (b) administering a second composition comprising an amount of antigen sufficient to modulate the immune response to an antigen wherein the antigen is linked to a polynucleotide comprising an immunostimulatory oligonucleotide. The modulation in the immune response should palliate the response or disorder. As described herein, it is understood that, in these embodiments, the amount of antigen administered is higher than that which would be administered in the absence of administering an IgE inhibitor (i.e. the amount of antigen administered is higher than that generally used in conventional desensitization therapy). In a preferred embodiment, the first composition comprises an anti-IgE antibody, preferably humanized, as described below. In addition to an anti-IgE antibody, the antigen of the second composition is linked to a polynucleotide comprising an immunostimulatory oligonucleotide (i.e. an ISS). This linkage may be covalent, non-covalent, or via other mechanisms, such as enacapulation or linkage to a platform molecule. In other embodiments, the first composition comprises an anti-IgE antibody, and the second composition comprises an antigen linked to a polynucleotide comprising or consisting of an ISS, preferably by conjugation.

In another embodiment, methods are provided for treating an IgE-associated disorder in a subject comprising administering to the subject an amount of a first composition that inhibits the activity of IgE sufficient to palliate the disorder; and administering to the subject an amount of a second composition comprising an immunostimulatory oligonucleotide sufficient to augment the activity of the first composition. In other embodiments, an antigen(s) is also administered. Generally, the first composition is administered in an amount sufficient to decrease the activity of IgE. These embodiments are particularly useful because administration of an ISS helps decrease and maintain the decrease in the IgE level, while enhancing a Th1 response. The IgE-associated disorder, as discussed below, includes, but is not limited to, allergy (any type of allergy); allergy-related disorders; and asthma.

In the methods of the invention, a composition is administered that inhibits the activity of IgE (i.e. the composition contains an agent(s) which inhibits IgE activity). Preferably, this inhibition is sufficient to avoid, or suppress the extent of, anaphylaxis upon administration and/or exposure to antigen.

In another embodiment, the invention provides methods for treating an IgE-associated disorder in an individual, comprising administering to the individual a first composition comprising an agent that inhibits anaphylaxis, or reduces the risk of analphylaxis, preferably an agent that inhibits IgE activity, wherein an effective amount of the first composition is an amount effective to inhibit anaphylaxis (or reduce the risk of analphylaxis), and a second composition comprising an ISS (or a polynucleotide comprising an immunostimulatory oliognucleotide), wherein an effective amount is an amount sufficient to enhance, or augment, the activity of the first composition. Preferably, antigen is also administered to the individual (either in the second composition or in a third composition).

In a preferred embodiment, the agent(s) in the first composition is an anti-IgE antibody, preferably humanized, as described below. In some embodiments, in addition to an anti-IgE antibody, antigen linked to a polynucleotide comprising an immunostimulatory oligonucleotide (i.e., ISS) is administered. This linkage may be covalent, non-covalent, or via other mechanisms, such as enacapulation or linkage to a platform molecule. In other embodiments, the first agent is an anti-IgE antibody, and the second composition comprises an antigen linked to ISS (or a polynucleotide comprising an ISS), preferably by conjugation.

### Compositions for inhibiting IgE activity

In the methods of the invention, a first composition is administered that inhibits IgE activity (i.e., contains an agent that inhibits IgE activity), and most preferably inhibits anaphylaxis (or lowers to eliminates the risk of anaphylaxis), particularly systemic anaphylaxis. Preferably, and as discussed below, an anti-IgE antibody is used, more preferably a humanized antibody.

Inhibitors of IgE activity are known in the art and, include, but are not limited to, anti-IgE antibodies, IgE binding fragments (including antibody fragments), receptors, or fragments thereof. For example, some inhibitors of IgE activity of the invention act by blocking the binding of IgE to its receptors on B cells, mast cells or basophils, either by blocking the receptor binding site on the IgE molecule or by blocking the IgE binding site on the receptor. Through the binding to IgE on the surface of B cells, an anti-IgE antibody may lead to the clonal elimination of the IgE-producing B cells and so, to a decrease in IgE production. Also, inhibitors of IgE activity also may act by binding soluble IgE and thereby removing it from circulation.

Inhibitors of IgE activity are known in the art and, include, but are not limited to, anti-IgE antibodies, antigen binding fragments, receptors, or fragments thereof. U.S. Patent 5,614,611 discloses humanized anti-IgE monoclonal antibodies specific for IgE-bearing B cells. By specifically binding to B cells and not to basophils or mast cells, these anti-IgE antibodies do not induce the release of histamine from basophils or mast cells.

U.S. Patent 5,449,760 describes anti-IgE antibodies that bind soluble IgE but not IgE on the surface of B cells or basophils. Antibodies such as these bind to soluble IgE and inhibit IgE activity by, for example, blocking the IgE receptor binding site, by blocking the antigen binding site and/or by simply removing the IgE from circulation. Additional anti-IgE antibodies and IgE-binding fragments derived from the anti-IgE antibodies are described in U.S. Patent 5,656,273. U.S. Patent 5,543,144 describes anti-IgE antibodies that bind soluble IgE and membrane-bound IgE on IgE-expressing B cells but not to IgE bound to basophils.

Another method of inhibiting IgE activity is to remove circulating IgE using apheresis, whereby plasma or serum is passed over an affinity column, which selectively removes antibody. The degree of selectivity can be determined by the nature of the affinity column. For example, an affinity column could be devised in which an anti-IgE antibody (either polyclonal or monoclonal) is cross-linked to the column matrix.

### Preparation of anti-IgE antibodies

As described herein, inhibitors of IgE activity include, but are not limited to, anti-IgE antibodies and anti-IgE receptor antibodies.

For the production of anti-IgE antibodies, human IgE for immunization may be purified from human serum, for example. Alternatively, human IgE may be produced by culturing an IgE-producing cell line, for example, the cell line U266, ATCC number CRL8033. Human IgE may be purified by affinity chromatography, as known in the art. For example, mouse monoclonal antibodies specific for human IgE conjugated to a suitable matrix may provide an IgE-specific immunoadsorbent. After the IgE preparation has contacted the immunoadsorbent, the adsorbed IgE can be eluted in substantially pure form from the immunoadsorbent. Such a preparation of human IgE can be used as an immunogen for the production of anti-IgE antibodies.

Polyclonal antibodies can be raised by administration of the immunogenic conjugate to a mammalian host, usually mixed with an adjuvant. The immunogen is conveniently prepared for injection by rehydrating lyophilized immunogen to form a solution or suspension. Preferred adjuvants are water-in-oil immersions, particularly Freund's complete adjuvant for the first administration, and Freund's incomplete adjuvant for booster doses. The preparation is typically administered in a variety of sites, and typically in two or more doses over a course of at least 4 weeks. Serum is harvested and tested for the presence of specific antibody using a hapten-protein conjugate or other competitive binding compound for the analyte in a standard immunoassay or precipitation reaction.

Polyclonal antisera will typically contain antibodies not reactive with the analyte or having undesired cross-reactivities. Methods for purifying specific antibodies from a polyclonal antiserum are known, particularly affinity purification using a column of analyte conjugated to a solid phase. The antiserum is passed over the column, the column is washed, and the antibody is eluted with a mild denaturing buffer such as 0.1 M glycine, 0.2 M NaCl, pH 2.5. If the antiserum is passed over the column in a buffer containing potential interfering substances, then the bound and eluted fraction will be enriched for antibodies that don't cross-react.

Preferably, anti-IgE antibodies for use in this invention are monoclonal. Monoclonal antibodies can be prepared by a number of different techniques known in the art. For hybridoma technology, the reader is referred generally to Harrow & Lane (1988), U.S. Patent Nos. 4,491,632, 4,472,500, and 4,444,887, and *Methods in Enzymology,* 73B:3 (1981). The most common way to produce monoclonal antibodies is to immortalize and clone a splenocyte or other antibody-producing cell recovered from an immunized animal. The clone is immortalized by a procedure such as fusion with a non-producing myeloma, by transfecting with Epstein Barr Virus, or transforming with oncogenic DNA. The treated cells are cloned and cultured, and clones are selected that produce antibody of the desired specificity. Specificity testing is performed on culture supernatants by a number of techniques, such as using the immunizing antigen as the detecting reagent in an immunoassay. A supply of monoclonal antibody from the selected clone can then be purified from a large volume of culture supernatant, or from the ascites fluid of suitably prepared host animals injected with the clone. The antibody can be tested for activity as raw supernatant or ascites, and is optionally purified using standard biochemical preparation techniques such as ammonium sulfate precipitation, ion exchange chromatography, and gel filtration chromatography.

Alternative methods for obtaining monoclonal antibodies involve contacting an immunocompetent cell or viral particle with a the desired analyte or an analyte-protein complex in vitro. In this context, "immunocompetent" means that the cell or particle is capable of expressing an antibody specific for the antigen without further genetic rearrangement, and can be selected from a cell mixture by presentation of the antigen. Immunocompetent eukaryotic cells can be harvested from an immunized mammalian donor, or they can be harvested from an unimmunized donor and prestimulated in vitro by culturing in the presence of immunogen and immunostimulatory growth factors. Cells of the desired specificity can be selected by contacting with the immunogen under culture conditions that result in proliferation of specific clones but not non-specific clones. Immunocompetent phage can be constructed to express immunoglobulin variable region segments on their surface. See Marks et al. (1996) *N. Engl. J. Med*. 335:730; WO patent applications 94/13804, 92/01047, 90/02809; and McGuinness et al. (1996) *Nature Biotechnol*. 14:1149. Phage of the desired specificity can be selected, for example, by adherence to a hapten-protein complex attached to a solid phase, and then amplified in *E*. *coli*.

Antibodies can also be prepared by bioengineering techniques. Amino acid sequence of heavy and light chain variable regions of the desired specificity are obtained from prototype antibody molecules and optionally modified, for example, for purposes of humanization. Polynucleotides encoding them are then synthesized or cloned, operatively linked to transcription and translation elements, and then expressed in a suitable host cell. See, for example, U.S. Patent Nos. 5,225,539 and 5,693,761.

Even more preferably, antibodies used in the invention are "humanized" or chimeric. Humanized antibodies comprise a variable or antigen binding (hypervariable or complementarity determining) regions derived from an animal (e.g., mouse) antibody and the remaining regions derived from a human antibody. Humanized antibodies are generally less immunogenic in human than nonhuman antibodies. Methods for producing humanized antibodies are described in the art. See, for example, U.S. Patent No. 5,449,760 and Better et al. (1988) *Science* 240:1041-1043.

The amount of antibody (or any IgE inhibitor) to be administered may be determined empirically; further, suggested doses are typically found in the publications describing the antibodies. Examples of effective amounts of anti-IgE antibodies can be found, for example, in U.S. Patents 5,543,144 and 5,656,273. That an appropriate amount has been administered may be indicated, for example, by measuring IgE levels and/or monitoring symptoms.

### IgE binding fragments

Inhibitors of IgE activity include, but are not limited to, IgE binding fragments. The term "IgE binding fragment" as used in this disclosure encompasses not only intact immunoglobulin molecules, but also such fragments and derivatives (including recombinant derivatives) of immunoglobulin molecules or T cell receptors with the desired specificity. An antigen binding fragment can consist of a single polypeptide chain (such as an scFv), multiple polypeptides linked by disulfide bonds (such as an intact immunoglobulin) or multiple peptides that are non-covalently associated (such as Fv fragments). Antigen binding fragments typically contain two opposing variable domains, each from an immunoglobulin or T cell receptor, but single variable domains of sufficient affinity can be found that bind antigen on their own.

Antibody fragments can be prepared by methods of standard protein chemistry, such as subjecting the antibody to cleavage with a proteolytic enzyme like pepsin, papain, or trypsin; and reducing disulfide bonds with such reagents as dithiothreitol. Examples include Fab fragments (comprising V_{H}-C_{H1} and V_{L}-C_{L} domains), F(ab)₂ fragments, Fd fragments (comprising V_{H}-C_{H1}-C_{H2} and V_{L}-C_{L} domains), Fv fragments (comprising V_{H} and V_{L} domains), and isolated heavy or light chain fragments with antigen binding activity. Genetically engineered variants of intact immunoglobulin can be produced by obtaining a polynucleotide encoding the antibody, and applying the general methods of molecular biology to splice encoding sequences or introduce mutations and translate the variant. Engineered variants of particular interest include chimeric and humanized antibodies, Fab-like fragments, single-chain variable region fragments (scFv, comprising heavy and light chain variable regions joined by a peptide linker), and diabodies (peptides comprising two variable regions that dimerize to form a bivalent antigen binding peptide).

Antigen binding activity can be determined by any suitable binding assay in which the antigen and the candidate fragment are combined under conditions that permit complexes to occur - typically, an isotonic buffer at physiological pH. The formation of stable complexes is determined, for example, by physicochemical means (such as a biosensor) or using a suitable label (such as a radioisotope or enzyme label) attached to one of the reacting components. The presence of stable complexes correlates with binding activity. Overall avidity between antigen and peptide is preferably at least about 10⁸M⁻¹, more preferably at least about 10¹⁰M⁻¹, and still more preferably at least about 10¹²M⁻¹. Antigen binding fragments are typically developed by obtaining an antibody prototype of the desired specificity, producing a fragment or otherwise adapting the structure, and then retesting for binding activity. Subfragmentation or modification can continue as long as antigen binding activity remains.

As is understood in the art, the amount of IgE binding fragments (or any IgE inhibitor) may be determined empirically. That an appropriate amount has been administered may be indicated, for example, by measuring IgE levels and/or monitoring symptoms.

### Antigen Immunotherapy

In some embodiments of the invention, antigen (i.e., a composition comprising an antigen(s)) is administered. Because the antigen(s) is administered in the context of administration of a composition that inhibits IgE activity (especially one that inhibits anaphylaxis upon administration and/or exposure to antigen), it is understood that the amount of antigen administered is higher than the amount that would be administered in the absence of a composition that inhibits IgE activity. Accordingly, such amounts are greater than the "maximum tolerated dose" (MTD) of conventional allergy immunotherapy, which does not encompass or contemplate using an IgE inhibitor. As is known in the art, an MTD is the highest does not inducing a systemic anaphylactic reaction. Alternatively, the amount of antigen given is less than the MTD for a subject who is receiving treatment according to the invention.

Accordingly, in some embodiments, antigen is administered in amounts of at least about 1.25, at least about 1.5, at least about 1.75, at least about 2.00 times higher than the amount that would have been given, or would be given, during conventional desensitization therapy (i.e., in the absence of administering a composition that inhibits IgE activity). In other embodiments, antigen is administered in amounts of at least about 5, at least about 7, at least about 10, at least about 12, at least about 15, at least about 20 times higher than the amount that would have been given, or would be given, during conventional desensitization therapy. In some embodiments, an initial dose of antigen is any of the following: about 0.25, about 0.5, about 1.0, about 2.0, about 5.0, about 10, about 15, about 20 µg. In some embodiments, a final, or endpoint, dose is any of the following: about 25, about 40, about 50, about 75, about 100, about 125, about 150, about 175, about 200 µg. The amount of antigen administered could be effected in higher concentrations and/or higher volumes. Accordingly, the "amount" of antigen could be increased in terms of concentration. It is understood that, in addition to administering higher than conventional dosages, it is also possible (but not required) to more rapidly increase the sequential dosages.

Dosing protocols are known in the art. See, e.g., Weber (1997); Fornadley (1998) *Otolaryngology Clinics North America* 31:111-127; *Remington's Pharmaceutical Sciences* 19th Ed. Mack Publising (1995), Chapter 82. Dosages for each antigen depend upon the particular antigen as well as the individual receiving therapy, and further are at some discretion of the individual practitioner. Generally, allergen immunotherapy is administered weekly with increasing doses, and the amount of antigen administered is in the microgram range. The amount of antigen is increased until alleviation (to elimination) of symptoms is observed or adverse symptoms are observed. Alternatively, so-call "rush" therapy involves initially administering clusters of administrations (such as several in a day, once a week), followed by weekly administrations. The therapy is given perennially, with a maintenance dose given at 2 to 4 week intervals. Over time, maintenance doses may be scheduled even less frequently, such as every 6 to 8 weeks.

In the methods of the invention, varying amounts of antigen may be used, as long as the dosage does not induce systemic anaphylaxis (i.e., MTD). Dosages lower than MTD may also be used.

Many antigens are known and have been isolated, often by recombinant techniques. Table 1 shows a list of allergens that may be used.

**TABLE 1 RECOMBINANT ALLERGENS**

| **Group** | **Allergen** | **Reference** |
|---|---|---|
| **ANIMALS:** | | |
| **CRUSTACEA** | | |
| Shrimp/lobster | tropomyosin | Leung et al. J Allergy Clin Immunol, 1996, 98: 954-61 |
| | Pan s I | Leung et al. Mol Mar Biol Biotechnol, 1998, 7:12-20 |

| **INSECTS** | | |
|---|---|---|
| Ant | Sol i 2 (venom) | Schmidt et al. J Allergy Clin Immunol., 1996, 98:82-8 |
| Bee | phospholipase A2 (PLA) | Muller et al. J Allergy Clin Immunol, 1995, 96:395-402 |
| | | Forster et al. J Allergy Clin Immunol, 1995, 95:1229-35 |
| | | Muller et al. Clin Exp Allergy, 1997, 27:915-20 |
| | | |
| | Hyaluronidase (Hya) | Soldatova et al. J Allergy Clin Immunol, 1998, 101:691-8 |
| Cockroach | Bla g Bd9OK | Helm et al. J Allergy Clin Immunol, 1996,98:172-80 |
| | | |
| | Bla g 4 (a calycin) | Vailes et al. J Allergy Clin Immunol, 1998, 101:274-80 |
| | | |
| | glutathione S-transferase | Arruda et al. J Biol Chem, 1997, 272:20907-12 |
| | | |
| | per a 3 | Wu et al. Mol Immunol, 1997, 34:1-8 |
| Dust mite | Der p 2 (major allergen) | Lynch et al. J Allergy Clin Immunol, 1998, 101:562-4 |
| | | Hakkaart et al. Clin Exp Allergy, 1998, 28:169-74 |
| | | Hakkaart et al. Clin Exp Allergy, 1998, 28:45-52 |
| | | Hakkaart et al. Int Arch Allergy Immunol, 1998, 115 |
| | | (2):150-6 |
| | | Mueller et al. J Biol Chem, 1997, 272:26893-8 |
| | Der p 2 variant | |
| | | Smith et al. J Allergy Clin Immunol, 1998, 101:423-5 |
| | Der f 2 | |
| | | Yasue et al. Clin Exp Immunol, 1998, 113:1-9 |
| | | Yasue et al. Cell Immunol, 1997, 181:30-7 |
| | Der p 10 | |
| | | Asturias et al. Biochim Biophys Acta, 1998, 1397:27-30 |
| | Tyr p 2 | |
| | | Eriksson et al. Eur J Biochem, 1998 |
| Hornet | Antigen 5 aka Dol m V (venom) | Tomalski et al. Arch Insect Biochem Physiol, 1993, 22:303-13 |
| Mosquito | Aed a I (salivary apyrase) | Xu et al. Int Arch Allergy Immunol, 1998, 115:245-51 |
| Yellow jacket | antigen 5, hyaluronidase, and phospholipase (venom) | King et al. J Allergy Clin Immunol, 1996, 98:588-600 |

| **MAMMALS** | | |
|---|---|---|
| Cat | Fel d I | Slunt et al. J Allergy Clin Immunol, 1995, 95:1221-8 - |
| | | Hoffmann et al. J Allergy Clin Immunol, 1997, 99: 227-32 |
| | | Hedlin Curr Opin Pediatr, 1995, 7:676-82 |
| Cow | Bos d 2 (dander; a lipocalin) | Zeiler et al. J Allergy Clin Immunol, 1997, 100:721-7 |
| | | Rautiainen et al. Biochem Bioph. Res Comm., 1998, 247:746-50 |
| | | |
| | β-lactoglobulin (BLG, major cow milk allergen) | Chatel et al. Mol Immunol, 1996, 33:1113-8 |
| | | Lehrer et al. Crit Rev Food Sci Nutr, 1996, 36:553-64 |
| Dog | Can f 1 and Can f 2, salivary lipocalins | Konieczny et al. Immunology, 1997, 92:577-86 |
| | | Spitzauer et al. J Allergy Clin Immunol, 1994, 93:614-27 |
| | | Vrtala et al. J Immunol, 1998, 160:6137-44 |
| Horse | Equ c 1 (major allergen, a lipocalin) | Gregoire et al. J Biol Chem, 1996, 271:32951-9 |
| Mouse | mouse urinary protein (MUP) | Konieczny et al. Immunology, 1997, 92:577-86 |

| **OTHER MAMMALIAN ALLERGENS** | | |
|---|---|---|
| Insulin | | Ganz et al. J Allergy Clin Immunol, 1990, 86:45-51 |
| | | Grammer et al. J Lab Clin Med, 1987, 109:141-6 |
| | | Gonzalo et al. Allergy, 1998, 53:106-7 |
| Interferons | interferon alpha 2c | Detmar et al. Contact Dermatis, 1989, 20:149-50 |
| **MOLLUSCS** | topomyosin | Leung et al. J Allergy Clin Immunol, 1996, 98 (5 Pt 1):954-61 |

| **PLANT ALLERGENS:** | | |
|---|---|---|
| Barley | Hor v 9 | Astwood et al. Adv Exp Med Biol, 1996, 409:269-77 |
| Birch | pollen allergen, Bet v 4 | Twardosz et al. Biochem Bioph. Res Comm., 1997, 23 9:197 |
| | rBet v 1 Bet v 2: (profilin) | Pauli et al. J Allergy Clin Immunol, 1996, 97:1100-9 |
| | | van Neerven et al. Clin Exp Allergy, 1998, 28:423-33 |
| | | Jahn-Schmid et al. Immunotechnology, 1996, 2:103-13 |
| | | Breitwieser et al. Biotechniques, 1996, 21:918-25 |
| | | Fuchs et al. J Allergy Clin Immunol, 1997, 100:3 56-64 |
| Brazil nut | globulin | Bartolome et al. Allergol Immunopathol, 1997,25:135-44 |
| Cherry | Pru a 1 (major allergen) | Scheurer et al. Mol Immunol, 1997, 34:619-29 |
| Corn | Zml3 (pollen) | Heiss et al. FEBS Lett, 1996, 381:217-21 |
| | | Lehrer et al. Int Arch Allergy Immunol, 1997, 113:122-4 |
| Grass | Phl p 1, Phl p 2, Phl p 5 (timothy grass pollen) | Bufe et al. Am J Respir Crit Care Med, 1998, 157:1269-76 |
| | | Vrtala et al. J Immunol Jun 15, 1998, 160:6137-44 |
| | | Niederberger et al. J Allergy Clin Immun., 1998, 101:258-64 |
| | Hol l 5 velvet grass pollen | Schramm et al. Eur J Biochem, 1998, 252:200-6 |
| | Bluegrass allergen | Zhang et al. J Immunol, 1993, 151:791-9 |
| | Cyn d 7 Bermuda grass | Smith et al. Int Arch Allergy Immunol, 1997, 114:265-71 |
| | Cyn d 12 (a profilin) | Asturias et al. Clin Exp Allergy, 1997, 27:1307-13 |
| | | Fuchs et al. J Allergy Clin Immunol, 1997, 100:356-64 |
| Juniper | Jun o 2 (pollen) | Tinghino et al. J Allergy Clin Immunol, 1998, 101:772-7 |
| Latex | Hev b 7 | Sowka et al. Eur J Biochem, 1998, 255:213-9 |
| | | Fuchs et al. J Allergy Clin Immunol, 1997, 100:3 56-64 |
| Mercurialis | Mer a I (profilin) | Vallverdu et al. J Allergy Clin Immunol, 1998, 101:3 63-70 |
| Mustard (Yellow) | Sin a I (seed) | Gonzalez de la Pena et al. Biochem Bioph. Res Comm., 1993, 190:648-53 |
| Oilseed rape | Bra r I pollen allergen | Smith et al. Int Arch Allergy Immunol, 1997, 114:265-71 |
| Peanut | Ara h I | Stanley et al. Adv Exp Med Biol, 1996, 409:213-6 |
| | | Burks et al. J Clin Invest, 1995, 96:1715-21 |
| | | Burks et al. Int Arch Allergy Immunol, 1995, 107:248-50 |
| Poa pratensis | Poa p9 | Parronchi et al. Eur J Immunol, 1996, 26:697-703 |
| | | Astwood et al. Adv Exp Med Biol, 1996, 409:269-77 |
| Ragweed | Amb a I | Sun et al. Biotechnology Aug, 1995, 13:779-86 |
| | | Hirschwehr et al. J Allergy Clin Immunol, 1998, 101:196-206 |
| | | Casale et al. J Allergy Clin Immunol, 1997, 100:110-21 |
| Rye | Lol p I | Tamborini et al. Eur J Biochem, 1997, 249:886-94 |
| Walnut | Jug r I | Teuber et al. J Allergy Clin lmmun., 1998, 101:807-14 |
| Wheat | allergen | Fuchs et al. J Allergy Clin Immunol, 1997, 100:356-64 |
| | | Donovan et al. Electrophoresis, 1993, 14:917-22 |

| **FUNGI:** | | |
|---|---|---|
| Aspergillus | Asp f 1, Asp f 2, Asp f3, Asp f 4, rAsp f 6 | Crameri et al. Mycoses, 1998, 41 Suppl 1:56-60 |
| | | Hemmann et al. Eur J Immunol, 1998, 28:1155-60 |
| | | Banerjee et al. J Allergy Clin Immunol, 1997, 99:821-7 |
| | | Crameri Int Arch Allergy Immunol, 1998, 115:99-114 |
| | | Crameri et al. Adv Exp Med Biol, 1996, 409:111-6 |
| | | Moser et al. J Allergy Clin Immunol, 1994, 93: 1-11 |
| | | |
| | Manganese superoxide dismutase (MNSOD) | Mayer et al. Int Arch Allergy Immunol, 1997, 113:213-5 |
| Blomia | allergen | Caraballo et al. Adv Exp Med Biol, 1996, 409:81-3 |
| Penicillinium | allergen | Shen et al. Clin Exp Allergy, 1997, 27:682-90 |
| Psilocybe | Psi c 2 | Homer et al. Int Arch Allergy Immunol, 1995, 107:298-300 |

### Administration of ISS

In some embodiments, the methods of the invention comprise administration of ISS (i.e., a polynucleotide comprising an ISS) in addition to administration of an IgE inhibitor (or anaphylaxis inhibitor), generally in an amount sufficient to augment the activity of the IgE inhibitor. As would be understood by one skilled in the art, this augmentation may be manifested in any of a number of ways, including, but not limited to, allowing the enhanced persistence of IgE suppression and/or increasing the reduction of IgE activity when compared to administering IgE inhibitor alone.

ISS are known and may readily be identified for immunostimulatory activity using standard methods in the art, such as measurement of cytokine and/or antibody production. Generally, ISS comprise the sequence 5'-C, G-3'. More particularly, ISS comprise the hexameric sequence 5', purine, purine, C, G, pyrimidine, pyrimidine-3'.

Preferred ISS comprises the general octameric sequence 5'-Purine, Purine, Cytosine, Guanine, Pyrimidine, Pyrimidine, Cytosine, (Cytosine or Guanine)-3'. Most preferably, the ISS comprises an octamer selected from the group consisting of: AACGTTCC, AACGTTCG, GACGTTCC, and GACGTTCG. In some embodiments, the ISS comprises the sequence TGACTGTGAACGTTCGAGATGA.

In some embodiments an ISS-antigen conjugate is administered. Making covalent and/or non-covalent linkages between polynucleotide and other moieties, such as peptides, employs standard techniques in the art.

The ISS, or the polynucleotide comprising an ISS, can be coupled with the immunomodulatory molecule portion of a conjugate in a variety of ways, including covalent and/or non-covalent interactions.

The link between the portions can be made at the 3' or 5' end of the ISS-containing polynucleotide, or at a suitably modified base at an internal position in the ISS. If the immunomodulatory molecule is a peptide and contains a suitable reactive group (e.g., an N-hydroxysuccinimide ester) it can be reacted directly with the N⁴ amino group of cytosine residues. Depending on the number and location of cytosine residues in the ISS, specific labeling at one or more residues can be achieved.

Alternatively, modified oligonucleosides, such as are known in the art, can be incorporated at either terminus, or at internal positions in the ISS-containing polynucleotide. These can contain blocked functional groups which, when deblocked, are reactive with a variety of functional groups which can be present on, or attached to, the immunomodulatory molecule of interest.

Where the immunomodulatory molecule is a peptide, this portion of the conjugate can be attached to the 3'-end of the ISS through solid support chemistry. For example, the ISS portion can be added to a polypeptide portion that has been pre-synthesized on a support. Haralambidis et al. (1990a) *Nucleic Acids Res.* 18:493-499; and Haralambidis et al. (1990b) *Nucleic Acids Res.* 18:501-505. Alternatively, the ISS can be synthesized such that it is connected to a solid support through a cleavable linker extending from the 3'-end. Upon chemical cleavage of the ISS from the support, a terminal thiol group is left at the 3'-end of the oligonucleotide (Zuckermann et al. (1987) *Nucleic Acids Res.* 15:5305-5321; and Corey et al. (1987) *Science* 238:1401-1403) or a terminal amine group is left at the 3'-end of the oligonucleotide (Nelson et al. (1989) *Nucleic Acids Res.* 17:1781-1794). Conjugation of the amino-modified ISS to amino groups of the peptide can be performed as described in Benoit et al. (1987) *Neuromethods* 6:43-72. Conjugation of the thiol-modified ISS to carboxyl groups of the peptide can be performed as described in Sinah et al. (1991) *Oligonucleotide Analogues: A Practical Approach,* IRL Press. Coupling of an oligonucleotide carrying an appended maleimide to the thiol side chain of a cysteine residue of a peptide has also been described. Tung et al. (1991) *Bioconjug. Chem.* 2:464-465.

The peptide portion of the conjugate can be attached to the 5'-end of the ISS-containing polynucleotide through an amine, thiol, or carboxyl group that has been incorporated into the oligonucleotide during its synthesis. Preferably, while the oligonucleotide is fixed to the solid support, a linking group comprising a protected amine, thiol, or carboxyl at one end, and a phosphoramidite at the other, is covalently attached to the 5'-hydroxyl. Agrawal et al. (1986) *Nucleic Acids Res.* 14:6227-6245; Connolly (1985) *Nucleic Acids Res.* 13:4485-4502; Kremsky et al. (1987) *Nucleic Acids Res.* 15:2891-2909; Connolly (1987) *Nucleic Acids Res.* 15:3131-3139; Bischoff et al. (1987) *Anal. Biochem.* 164:336-344; Blanks et al. (1988) *Nucleic Acids Res.* 16:10283-10299; and U.S. Patent Nos. 4,849,513, 5,015,733, 5,118,800, and 5,118,802. Subsequent to deprotection, the latent amine, thiol, and carboxyl functionalities can be used to covalently attach the oligonucleotide to a peptide. Benoit et al. (1987); and Sinah et al. (1991).

The peptide portion can be attached to a modified cytosine or uracil at any position in the ISS-containing polynucleotide. The incorporation of a "linker arm" possessing a latent reactive functionality, such as an amine or carboxyl group, at C-5 of the modified base provides a handle for the peptide linkage. Ruth, *4th Annual Congress for Recombinant DNA Research,* p. 123.

An ISS-immunomodulatory molecule conjugate can also be formed through non-covalent interactions, such as ionic bonds, hydrophobic interactions, hydrogen bonds and/or van der Waals attractions.

Non-covalently linked conjugates can include a non-covalent interaction such as a biotin-streptavidin complex. A biotinyl group can be attached, for example, to a modified base of an ISS. Roget et al. (1989) *Nucleic Acids Res.* 17:7643-7651. Incorporation of a streptavidin moiety into the peptide portion allows formation of a non-covalently bound complex of the streptavidin conjugated peptide and the biotinylated oligonucleotide.

Non-covalent associations can also occur through ionic interactions involving an ISS and residues within the immunomodulatory molecule, such as charged amino acids, or through the use of a linker portion comprising charged residues that can interact with both the oligonucleotide and the immunomodulatory molecule. For example, non-covalent conjugation can occur between a generally negatively-charged ISS and positively-charged amino acid residues of a peptide, e.g., polylysine and polyarginine residues.

Non-covalent conjugation between ISS and immunomodulatory molecules can occur through DNA binding motifs of molecules that interact with DNA as their natural ligands. For example, such DNA binding motifs can be found in transcription factors and anti-DNA antibodies.

The linkage of the ISS to a lipid can be formed using standard methods. These methods include, but are not limited to, the synthesis of oligonucleotide-phospholipid conjugates (Yanagawa et al. (1988) *Nucleic Acids Symp. Ser.* 19:189-192), oligonucleotide-fatty acid conjugates (Grabarek et al. (1990) *Anal. Biochem.* 185:131-135; and Staros et al. (1986) *Anal. Biochem.* 156:220-222), and oligonucleotide-sterol conjugates. Boujrad et al. (1993) *Proc. Natl. Acad. Sci. USA* 90:5728-5731.

The linkage of the oligonucleotide to an oligosaccharide can be formed using standard known methods. These methods include, but are not limited to, the synthesis of oligonucleotide-oligosaccharide conjugates, wherein the oligosaccharide is a moiety of an immunoglobulin. O'Shannessy et al. (1985) *J. Applied Biochem.* 7:347-355.

The linkage of a circular ISS to a peptide or antigen can be formed in several ways. Where the circular ISS is synthesized using recombinant or chemical methods, a modified nucleoside is suitable. Ruth (1991) in *Oligonucleotides and Analogues: A Practical Approach,* IRL Press. Standard linking technology can then be used to connect the circular ISS to the antigen or other peptide. Goodchild (1990) *Bioconjug. Chem*. 1:165. Where the circular ISS is isolated, or synthesized using recombinant or chemical methods, the linkage can be formed by chemically activating, or photoactivating, a reactive group (e.g. carbene, radical) that has been incorporated into the antigen or other peptide.

Additional methods for the attachment of peptides and other molecules to oligonucleotides can be found in U.S. Patent No. 5,391,723; Kessler (1992) "Nonradioactive labeling methods for nucleic acids" in Kricka (ed.) *Nonisotopic DNA Probe Techniques,* Academic Press; and Geoghegan et al. (1992) *Bioconjug. Chem.* 3:138-146.

An ISS may also be proximately associated with antigen(s) by (a) encapsidation (such as, for example, a liposome); (b) adsorption onto a surface; and (c) via a platform molecule (i.e., a synthetic or naturally-occurring molecule that contains sites which allow for attachment of ISS and antigen(s).

The ISS can be administered alone or in combination with other pharmaceutical and/or immunogenic and/or immunostimulatory agents and can be combined with a physiologically acceptable carrier thereof. The effective amount and method of administration of the particular ISS formulation can vary based on the individual patient and the stage of the disease and other factors evident to one skilled in the art. The route(s) of administration useful in a particular application are apparent to one of skill in the art. Routes of administration include but are not limited to topical, dermal, transdermal, transmucosal, epidermal parenteral, gastrointestinal, and naso-pharyngeal and pulmonary, including transbronchial and transalveolar. A suitable dosage range is one that provides sufficient ISS-containing composition to attain a tissue concentration of about 1-10 µM as measured by blood levels. The absolute amount given to each patient depends on pharmacological properties such as bioavailability, clearance rate and route of administration.

The present invention provides ISS-containing compositions suitable for topical application including, but not limited to, physiologically acceptable implants, ointments, creams, rinses and gels. Topical administration is, for instance, by a dressing or bandage having dispersed therein a delivery system, or by direct administration of a delivery system into incisions or open wounds. Creams, rinses, gels or ointments having dispersed therein an ISS-containing composition are suitable for use as topical ointments or wound filling agents.

Preferred routes of dermal administration are those which are least invasive. Preferred among these means are transdermal transmission, epidermal administration and subcutaneous injection. Of these means, epidermal administration is preferred for the greater concentrations of APCs expected to be in intradermal tissue.

Transdermal administration is accomplished by application of a cream, rinse, gel, etc. capable of allowing the ISS-containing composition to penetrate the skin and enter the blood stream. Compositions suitable for transdermal administration include, but are not limited to, pharmaceutically acceptable suspensions, oils, creams and ointments applied directly to the skin or incorporated into a protective carrier such as a transdermal device (so-called "patch"). Examples of suitable creams, ointments etc. can be found, for instance, in the Physician's Desk Reference.

For transdermal transmission, iontophoresis is a suitable method. Iontophoretic transmission can be accomplished using commercially available patches which deliver their product continuously through unbroken skin for periods of several days or more. Use of this method allows for controlled transmission of pharmaceutical compositions in relatively great concentrations, permits infusion of combination drugs and allows for contemporaneous use of an absorption promoter.

An exemplary patch product for use in this method is the LECTRO PATCH trademarked product of General Medical Company of Los Angeles, CA. This product electronically maintains reservoir electrodes at neutral pH and can be adapted to provide dosages of differing concentrations, to dose continuously and/or periodically. Preparation and use of the patch should be performed according to the manufacturer's printed instructions which accompany the LECTRO PATCH product; those instructions are incorporated herein by this reference.

For transdermal transmission, low-frequency ultrasonic delivery is also a suitable method. Mitragotri et al. (1995) *Science* 269:850-853. Application of low-frequency ultrasonic frequencies (about 1 MHz) allows the general controlled delivery of therapeutic compositions, including those of high molecular weight.

Epidermal administration essentially involves mechanically or chemically irritating the outermost layer of the epidermis sufficiently to provoke an immune response to the irritant. Specifically, the irritation should be sufficient to attract APCs to the site of irritation.

An exemplary mechanical irritant means employs a multiplicity of very narrow diameter, short tines which can be used to irritate the skin and attract APCs to the site of irritation, to take up ISS-containing compositions transferred from the end of the tines. For example, the MONO-VACC old tuberculin test manufactured by Pasteur Merieux of Lyon, France contains a device suitable for introduction of ISS-containing compositions.

The device (which is distributed in the U.S. by Connaught Laboratories, Inc. of Swiftwater, PA) consists of a plastic container having a syringe plunger at one end and a tine disk at the other. The tine disk supports a multiplicity of narrow diameter tines of a length which will just scratch the outermost layer of epidermal cells. Each of the tines in the MONO-VACC kit is coated with old tuberculin; in the present invention, each needle is coated with a pharmaceutical composition of ISS-containing composition. Use of the device is preferably according to the manufacturer's written instructions included with the device product. Similar devices which can also be used in this embodiment are those which are currently used to perform allergy tests.

Another suitable approach to epidermal administration of ISS is by use of a chemical which irritates the outermost cells of the epidermis, thus provoking a sufficient immune response to attract APCs to the area. An example is a keratinolytic agent, such as the salicylic acid used in the commercially available topical depilatory creme sold by Noxema Corporation under the trademark NAIR. This approach can also be used to achieve epithelial administration in the mucosa. The chemical irritant can also be applied in conjunction with the mechanical irritant (as, for example, would occur if the MONO-VACC type tine were also coated with the chemical irritant). The ISS can be suspended in a carrier which also contains the chemical irritant or coadministered therewith.

Another delivery method for administering ISS-containing compositions makes use of non-lipid polymers, such as a synthetic polycationic amino polymer. Leff (1997) *Bioworld* 86:1-2.

Parenteral routes of administration include but are not limited to electrical (iontophoresis) or direct injection such as direct injection into a central venous line, intravenous, intramuscular, intraperitoneal, intradermal, or subcutaneous injection. Compositions suitable for parenteral administration include, but are not limited to, pharmaceutically acceptable sterile isotonic solutions. Such solutions include, but are not limited to, saline and phosphate buffered saline for injection of the ISS-containing compositions.

Gastrointestinal routes of administration include, but are not limited to, ingestion and rectal. The invention includes ISS-containing compositions suitable for gastrointestinal administration including, but not limited to, pharmaceutically acceptable, powders, pills or liquids for ingestion and suppositories for rectal administration.

Naso-pharyngeal and pulmonary routes of administration include, but are not limited to, by-inhalation, transbronchial and transalveolar routes. The invention includes ISS-containing compositions suitable for by-inhalation administration including, but not limited to, various types of aerosols for inhalation, as well as powder forms for delivery systems. Devices suitable for by-inhalation administration of ISS-containing compositions include, but are not limited to, atomizers and vaporizers. Atomizers and vaporizers filled with the powders are among a variety of devices suitable for use in by-inhalation delivery of powders. See, e.g., Lindberg (1993) Summary of Lecture at Management Forum 6-7 December 1993 "Creating the Future for Portable Inhalers."

The methods of producing suitable devices for injection, topical application, atomizers and vaporizers are known in the art and will not be described in detail.

The choice of delivery routes can be used to modulate the immune response elicited. For example, IgG titers and CTL activities were identical when an influenza virus vector was administered via intramuscular or epidermal (gene gun) routes; however, the muscular inoculation yielded primarily IgG2A, while the epidermal route yielded mostly IgG1. Pertmer et al. (1996) *J. Virol*. 70:6119-6125. Thus, one of skill in the art can take advantage of slight differences in immunogenicity elicited by different routes of administering the compositions of the present invention.

The above-mentioned compositions and methods of administration are meant to describe but not limit the methods of the invention. The methods of producing the various compositions and devices are within the ability of one skilled in the art and are not described in detail here.

### Dosages

Dosages for IgE inhibitors (especially anti-IgE antibodies) and antigen have been discussed above, and generally involve empirical determinations well within the skill of the art. With respect to ISS, in general, a suitable dosage range is one that provides sufficient ISS-containing composition to attain a tissue concentration of about 1-10 µM as measured by blood levels. As with IgE inhibitor and antigen, suitable dosage for ISS may be determined empirically by one skilled in the art.

The compositions may be given in any order or simultaneously.

### Assessment of immune response

Analysis (both qualitative and quantitative) of the immune response to compositions of the present invention can be by any method known in the art, including, but not limited to, measuring antigen-specific antibody production, activation of specific populations of lymphocytes such as CD4⁺ T cells or NK cells, and/or production of cytokines such as IFN, IL-2, IL-4, or IL-12. Methods for measuring specific antibody responses include enzyme-linked immunosorbent assay (ELISA) and are well known in the art. Measurement of numbers of specific types of lymphocytes such as CD4⁺ T cells can be achieved, for example, with fluorescence-activated cell sorting (FACS). Cytotoxicity assays can be performed for instance as described in Raz et al. (1994) *Proc. Natl. Acad. Sci. USA* 91:9519-9523. Serum concentrations of cytokines can be measured, for example, by ELISA. These and other assays to evaluate the immune response to an immunogen are well known in the art. See, for example, *Selected Methods in Cellular Immunology* (1980) Mishell and Shiigi, eds., W.H. Freeman and Co. Assessment of immune response can also be subjective. For instance, a subject can report a decreased incidence or severity of allergic reactions to particular allergens.

### Kits and compositions

The present invention also provides kits and compositions suitable for use with the methods described herein.

Kits of the invention comprise any of the following compositions in suitable packaging: (a) an IgE inhibitor(s) and an antigen; (b) an antigen in higher concentration than is generally formulated for conventional desensitization therapy (see discussion below); (c) an IgE inhibitor(s) and one or more ISS; (d) an IgE inhibitor(s) and one or more ISS and an antigen; (e) an IgE inhibitor(s) and an ISS-Ag conjugate; (f) an IgE inhibitor(s) and an ISS proximately associated with antigen. The kits of the invention may optionally contain instructions for their use and/or any other suitable components.

The compositions of the invention are novel formulations in which the concentration of antigen(s) is higher than that (or those) used in current, conventional desensitization therapy. Accordingly, the amount of antigen per unit volume in the novel formulations of the invention is an amount effective to induce desensitization to the antigen (which is generally accomplished over a series of increasing dosages) while not inducing anaphylaxis. The amounts of antigen(s) in the novel formulations of the invention are significantly higher than, and not suggested by, the formulations of the prior art. In some embodiments, the concentrations are any of the following: about 2, about 3, about 5, about 10, about 15, about 20, about 25, about 50, about 75, about 100 times more concentrated than the formulations used in conventional therapy. Concentrations of antigen formulations currently in use are known in the art and need not be described herein.

The invention also provides compositions comprising a composition or agent that inhibits the activity of IgE and an immunostimulatory oligonucleotide (ISS).

Generally, the compositions of the invention preferably also comprise a pharmaceutically acceptable excipient. As is well known in the art, a pharmaceutically acceptable excipient is a relatively inert substance that facilitates administration of a pharmacologically effective substance. For example, an excipient can give form or consistency, or act as a diluent. Suitable excipients include but are not limited to stabilizing agents, wetting and emulsifying agents, salts for varying osmolarity, encapsulating agents, buffers, and skin penetration enhancers. Excipients as well as formulations for parenteral and nonperenteral drug delivery are set forth in *Remington's Pharmaceutical Sciences* 19th Ed. Mack Publishing (1995).

Other formulations include suitable delivery forms known in the art including, but not limited to, carriers such as liposomes. Mahato et al. (1997) *Pharm. Res.* 14:853-859. Liposomal preparations include, but are not limited to, cytofectins, multilamellar vesicles and unilamellar vesicles.

Generally, these compositions are formulated for administration by injection (e.g., intraperitoneally, intravenously, subcutaneously, intramuscularly, *etc*.). Accordingly, these compositions are preferably combined with pharmaceutically acceptable vehicles such as saline, Ringer's solution, dextrose solution, and the like. The particular dosage regimen, *i.e.,* dose, timing and repetition, will depend on the particular individual and that individual's medical history.

Other formulations include suitable delivery forms known in the art including, but not limited to, carriers such as liposomes, Mahato et al. (1997) *Pharm. Res*. 14:853-859. Liposomal preparations include, but are not limited to, cytofectins, multilamellar vesicles and unilamellar vesicles.

In some embodiments, more than one antigen(s) may be present in a composition. Such compositions may contain at least one, at least two, at least three, at least four, at least five different antigen(s). Such "cocktails", as they are often denoted in the art, may be particularly useful in treating individuals who, for example, are allergic to more than one allergen.

## Claims

1. A first composition that inhibits the activity of IgE sufficient to decrease the activity of IgE in a subject, wherein the first composition comprises an anti-IgE-antibody and a second composition comprising an amount of an antigen sufficient to modulate the immune response to the antigen wherein the antigen is linked to a polynucleotide comprising an immunostimulatory oligonucleotide, for use in combination in treating an allergic response to the antigen or allergy-related disorder during antigen-specific immunotherapy of the subject.

2. A first composition that inhibits the activity of IgE sufficient to palliate an IgE associated disorder, wherein the first composition comprises an anti-IgE antibody and a second composition comprising an immunostimulatory oligonucleotide sufficient to augment the activity of the first composition, for use in combination in treating a subject having the IgE associated disorder.

3. The compositions according to claim 2, wherein the IgE associated disorder is an allergy or allergy-related disorder, or a parasite infection.

4. The compositions according to claim 3, wherein the anti-IgE antibody is a humanized murine antibody.

5. The compositions according to any one of claims 2, 3 or 4, wherein the first composition is administered before the second composition, the second composition is administered before the first composition, or the first composition is administered with the second composition.

6. The compositions of any one of claims 2 to 5, for treating an allergic response or allergy-related disorder to an allergen during antigen-specific immunotherapy, further comprising a third composition comprising an amount of the antigen sufficient to induce desensitization to the allergen.

7. The compositions according to claim 6 wherein the second composition and third composition comprise a single composition comprising an iummunostimulatory oligonucleotide conjugated to the antigen.

8. A first composition comprising an agent that inhibits anaphylaxis in an amount effective to inhibit anaphylaxis, wherein the agent in the first composition is an anti-IgE antibody and a second composition comprising a polynucleotide comprising an immunostimulatory oligonucleotide in an amount sufficient to enhance the activity of the first composition, for use in combination in treating an IgE-associated disorder in an individual.

9. The compositions of claim 8, further comprising an antigen.

10. The compositions of claim 9, wherein the antigen is linked to the polynucleotide.

11. A composition comprising a composition that inhibits the activity of IgE, wherein the composition comprises an anti-IgE antibody and an immunostimulatory oligonucleotide.

12. A kit comprising:
(a) a composition that inhibits the activity of IgE, wherein the composition comprises an anti-IgE antibody; and
(b) an immunostimulatory oligonucleotide;
in suitable packaging.

## Patentansprüche

1. Erste Zusammensetzung, welche die Aktivität von IgE ausreichend hemmt, um die Aktivität von IgE in einem Individuum zu vermindern, wobei die erste Zusammensetzung einen Anti-IgE-Antikörper und eine zweite Zusammensetzung, umfassend eine ausreichende Menge eines Antigens, um die Immunantwort auf das Antigen zu modulieren, wobei das Antigen an ein Polynukleotid geknüpft ist, umfassend ein immunstimulatorisches Oligonukleotid, umfasst, zur Verwendung in Kombination in der Behandlung einer allergischen Reaktion auf das Antigen oder einer Allergie-bezogenen Störung während einer Antigen-spezifischen Immuntherapie des Individuums.

2. Erste Zusammensetzung, welche die Aktivität von IgE ausreichend hemmt, um eine IgE-bezogene Störung abzumildern, wobei die erste Zusammensetzung einen Anti-IgE-Antikörper und eine zweite Zusammensetzung, umfassend ausreichend immunstimulatorisches Oligonukleotid, um die Aktivität der ersten Zusammensetzung zu erhöhen, umfasst, zur Verwendung in Kombination in der Behandlung eines Individuums mit einer IgE-bezogenen Störung.

3. Zusammensetzungen nach Anspruch 2, wobei die IgE-bezogene Störung eine Allergie oder Allergie-bezogene Störung oder eine Parasiteninfektion ist.

4. Zusammensetzungen nach Anspruch 3, wobei der Anti-IgE-Antikörper ein humanisierter muriner Antikörper ist.

5. Zusammensetzungen nach einem der Ansprüche 2, 3 oder 4, wobei die erste Zusammensetzung vor der zweiten Zusammensetzung verabreicht wird, die zweite Zusammensetzung vor der ersten Zusammensetzung verabreicht wird, oder die erste Zusammensetzung mit der zweiten Zusammensetzung verabreicht wird.

6. Zusammensetzungen nach einem der Ansprüche 2 bis 5, zur Behandlung einer allergischen Reaktion oder Allergie-bezogenen Störung auf ein Allergen während einer Antigen-spezifischen Immuntherapie, außerdem umfassend eine dritte Zusammensetzung, die eine ausreichende Menge des Antigens umfasst, um eine Desensibilisierung gegen das Antigen herbeizuführen.

7. Zusammensetzungen nach Anspruch 6, wobei die zweite Zusammensetzung und die dritte Zusammensetzung eine einzige Zusammensetzung darstellen, umfassend ein immunstimulatorisches Oligonukleotid in Konjugation an das Antigen.

8. Erste Zusammensetzung, umfassend ein Agens, das eine Anaphylaxe hemmt, in einer wirksamen Menge zur Hemmung einer Anaphylaxe, wobei das Agens in der ersten Zusammensetzung ein Anti-IgE-Antikörper ist, und eine zweite Zusammensetzung, umfassend ein Polynukleotid, das ein immunstimulatorisches Oligonukleotid in einer ausreichenden Menge umfasst, um die Aktivität der ersten Zusammensetzung zu erhöhen, zur Verwendung in Kombination in der Behandlung einer IgE-bezogenen Störung in einem Individuum.

9. Zusammensetzungen nach Anspruch 8, außerdem umfassend ein Antigen.

10. Zusammensetzungen nach Anspruch 9, wobei das Antigen an das Polynukleotid geknüpft ist.

11. Zusammensetzung, umfassend eine Zusammensetzung, welche die Aktivität von IgE hemmt, wobei die Zusammensetzung einen Anti-IgE-Antikörper und ein immunstimulatorisches Oligonukleotid umfasst.

12. Kit, umfassend:
(a) eine Zusammensetzung, welche die Aktivität von IgE hemmt, wobei die Zusammensetzung einen Anti-IgE-Antikörper umfasst; und
(b) ein immunstimulatorisches Oligonukleotid;
in einer geeigneten Packung.

## Revendications

1. Première composition qui inhibe l'activité de IgE suffisamment pour diminuer l'activité de IgE chez un sujet, dans laquelle la première composition comprend un anticorps anti-IgE et deuxième composition comprenant une quantité d'un antigène suffisante pour moduler la réponse immunitaire à l'antigène, dans laquelle l'antigène est lié à un polynucléotide comprenant un oligonucléotide immunostimulateur, destinées à être utilisées en combinaison pour traiter une réponse allergique à l'antigène ou un trouble associé à une allergie pendant une immunothérapie spécifique à l'antigène du sujet.

2. Première composition qui inhibe l'activité de IgE suffisamment pour palier un trouble associé à IgE, dans laquelle la première composition comprend un anticorps anti-IgE et deuxième composition comprenant un oligonucléotide immunostimulateur suffisant pour augmenter l'activité de la première composition, destinées à être utilisées en combinaison pour traiter un sujet ayant le trouble associé à IgE.

3. Compositions selon la revendication 2, dans lesquelles le trouble associé à IgE est une allergie ou un trouble associé à une allergie, ou une infection parasitaire.

4. Compositions selon la revendication 3, dans lesquelles l'anticorps anti-IgE est un anticorps murin humanisé.

5. Compositions selon l'une quelconque des revendications 2, 3 et 4, dans lesquelles la première composition est administrée avant la deuxième composition, la deuxième composition est administrée avant la première composition, ou la première composition est administrée avec la deuxième composition.

6. Compositions selon l'une quelconque des revendications 2 à 5, pour traiter une réponse allergique ou un trouble associé à une allergie à un allergène pendant une immunothérapie spécifique à l'antigène, comprenant de plus une troisième composition comprenant une quantité de l'antigène suffisante pour induire une désensibilisation à l'allergène.

7. Compositions selon la revendication 6, dans lesquelles la deuxième composition et la troisième composition comprennent une composition unique comprenant un oligonucléotide immunostimulateur conjugué à l'antigène.

8. Première composition comprenant un agent qui inhibe une anaphylaxie selon une quantité efficace pour inhiber une anaphylaxie, dans laquelle l'agent dans la première composition est un anticorps anti-IgE et deuxième composition comprenant un polynucléotide comportant un oligonucléotide immunostimulateur selon une quantité suffisante pour accentuer l'activité de la première composition, destinées à être utilisées en combinaison pour traiter un trouble associé à IgE chez un individu.

9. Compositions selon la revendication 8, comprenant de plus un antigène.

10. Compositions selon la revendication 9, dans lesquelles l'antigène est lié au polynucléotide.

11. Composition comprenant une composition qui inhibe l'activité de IgE, la composition comprenant un anticorps anti-IgE et un oligonucléotide immunostimulateur.

12. Kit comprenant :
(a) une composition qui inhibe l'activité de IgE, dans laquelle la composition comprend un anticorps anti-IgE ; et
(b) un oligonucléotide immunostimulateur ;
dans un emballage approprié.
